# EUROPEAN PATENT APPLICATION

(11) **EP 3 252 631 A1**
(43) Date of publication of application: **06.12.2017**
(21) Application number: 17171228.4
(22) Date of filing: 16.05.2017
(51) Int. Cl.: G06F 19/00, G01G 19/44

(54) **TREATMENT SYSTEM INCLUDING MASSAGE MACHINE**

(30) Priority: 03.06.2016 JP 2016111519
(71) Applicant: Family Inada Co., Ltd., Osaka 532-0004 (JP)
(72) Inventor: INADA, Nichimu, Osaka, Osaka 532-0004 (JP); NOTSU, Yuji, Saihaku-gun, Tottori 689-3224 (JP); MORITOMO, Atsushi, Saihaku-gun, Tottori 689-3224 (JP)
(74) Representative: Horn Kleimann Waitzhofer Patentanwälte PartG mbB

(57) **Abstract**

An object is to provide a treatment system including a massage machine, in which when setting a target value related to a weight, a user can set an appropriate target value instead of setting an immoderate target value. A massage machine 10 has a massage portion 15 performing a massage with respect to a user, and a control unit 16 controlling the massage portion 15. There are provided setting means 30 for setting a numerical value which includes weight information of the user to be set as a target value, and a notification portion 31. The control unit 16 compares the target value and a predetermined reference value with each other and causes the notification portion 31 to urge the user to change the target value in a case where a predetermined condition is satisfied.

## Description

### TECHNICAL FIELD

The present invention relates to a treatment system including a massage machine.

### BACKGROUND ART

In the related art, a user has treated each site of the body by using a massage machine. In addition, for his/her own health management, the user has performed health management using a measurement instrument, such as weight management using a weight scale. In this manner, generally, the body treatment using the massage machine and the health management using the measurement instrument have been separately performed.

Meanwhile, there is a known massage machine in which a weight scale is built in a chair as an attachment and a weight can be taken by only having a seat (for example, refer to PTL 1).

In addition, as another technology in the related art, there is a known weight scale in which a target weight value or a target achievement date of a user can be set (for example, refer to PTL 2).

### RELATED ART DOCUMENT

### PATENT DOCUMENT

Patent Document 1: JP-A-2001-269381
Patent Document 2: Japanese Patent No. 3722678

### SUMMARY OF THE INVENTION

### PROBLEM THAT THE INVENTION IS TO SOLVE

PTL 1 discloses a massage machine which takes a measurement with a built-in weight scale and displays past histories and the like as a result. In the massage machine, a user sets a target weight or a target period. Accordingly, there is a problem in that the user cannot check the current state with respect to the target. In addition, PTL 2 discloses a weight scale in which a target weight value or a target achievement date of a user can be set. However, there is a problem in that the user cannot check whether or not the target weight value or the target achievement date is an appropriate setting value. Therefore, the present invention has been made in order to solve the foregoing problems and aims to provide a treatment system including a massage machine in which when setting a target value related to a weight, a user can set an appropriate target value instead of setting an immoderate target value.

### MEANS FOR SOLVING THE PROBLEM

According to the present invention, there is provided a massage machine which has a massage portion performing a massage with respect to a user, and a control unit controlling the massage portion. There are provided setting means for setting a numerical value which includes weight information of the user as a target value, and a notification portion. The control unit compares the target value and a predetermined reference value with each other and causes the notification portion to urge the user to change the target value in a case where a predetermined condition is satisfied.

According to the configuration, in a case where the user sets the target value related to a weight, if the target value is an immoderate value, the user is urged to change the target value. Thus, the user can set an appropriate target value instead of setting an immoderate target value.

In addition, it is preferable that the control unit compares the target value and the predetermined reference value with each other and prohibits the set target value from being set in a case where the predetermined condition is satisfied.

According to the configuration, the user cannot set an immoderate target value and can set only an appropriate target value.

In addition, it is preferable to further include input means for inputting user related information. It is preferable that the reference value is set based on the user related information.

According to the configuration, the reference value changes in accordance with the user related information. Therefore, the user can individually set a suitable reference value.

In addition, it is preferable that the numerical value is a weight, a BMI, and a body fat percentage or a body fat quantity. It is preferable for the predetermined condition to require that the target value is less than the reference value.

According to the configuration, the user can set a more appropriate target value.

In addition, according to the present invention, there is provided a massage machine which has a massage portion performing a massage with respect to a user, and a control unit controlling the massage portion. There are provided setting means for setting a current numerical value which includes weight information of the user to be set as a current value, a targeted numerical value which includes weight information of the user to be set as a target value, and a period or a time limit for attaining the target value; and a notification portion. The control unit causes the notification portion to urge the user to change at least one of the target value and the period or the time limit in a case where a relationship between a difference of the current value and the target value, and the period or the time limit satisfies a predetermined condition.

According to the configuration, in a case where the user sets the target value related to a weight and the period or the time limit, if the target value and the period or the time limit is an immoderate value, the user is urged to change the target value and the period or the time limit. Thus, the user can set an appropriate target value and an appropriate period or an appropriate time limit instead of setting an immoderate target value and an immoderate period or an immoderate time limit.

In addition, it is preferable that the control unit prohibits at least one of the target value and the period or the time limit from being set in a case where the relationship between the difference of the current value and the target value, and the period or the time limit satisfies the predetermined condition.

According to the configuration, the user cannot set an immoderate target value and an immoderate period or an immoderate time limit and can set only an appropriate target value and an appropriate period or an appropriate time limit.

In addition, it is preferable that the numerical value is a weight, a BMI, and a body fat percentage or a body fat quantity. It is preferable for the predetermined condition to require that an average change rate calculated based on the difference of the current value and the target value, and the period or the time limit exceeds a predetermined change rate.

According to the configuration, the user can set a more appropriate target value and a more appropriate period or a more appropriate time limit.

In addition, it is preferable to further include input means for inputting user related information. It is preferable that the change rate is set based on the user related information.

According to the configuration, the change rate changes in accordance with the user related information. Therefore, the user can individually set a suitable change rate.

In addition, according to the present invention, there is provided a massage machine which has a massage portion performing a massage with respect to a user, and a control unit controlling the massage portion. There are provided input means for inputting user related information, setting means for setting a current numerical value which includes weight information of the user to be set as a current value; and a notification portion. The control unit calculates a plurality of target value and a plurality of periods or a plurality of time limits for attaining the target values based on the information and the current value, draws out a plurality of choices constituted by the plurality of target values and the plurality of periods or the plurality of time limits, can causes the notification portion to issue a notification.

According to the configuration, the user can set a target suitable for himself/herself from the plurality of choices.

In addition, it is preferable that the notification portion issues a notification of target information indicating a relationship among the current value, the target value, and the period or the time limit.

According to the configuration, the user can be informed of the target information.

### ADVANTAGE OF THE INVENTION

According to the present invention, when setting a target value related to a weight, a user can set an appropriate target value instead of setting an immoderate target value.

### BRIEF DESCRIPTION OF THE DRAWINGS

Fig. 1 is a view illustrating the overall configuration of a treatment system according to an embodiment of the present invention.
Fig. 2 is a perspective front view of a massage machine illustrated in Fig. 1.
Fig. 3 is a front view of a massage unit.
Fig. 4 is a block diagram of the massage machine.
Fig. 5 is a front view illustrating an example of a screen for setting a target weight as a target value displayed in a screen of a controller illustrated in Fig. 2.
Fig. 6 is a front view illustrating another example of the screen for setting a target weight as a target value displayed in the screen of the controller illustrated in Fig. 2.
Fig. 7 is a front view illustrating an example of a screen for inputting user related information displayed in the screen of the controller illustrated in Fig. 2.
Fig. 8 is a front view illustrating an example of a screen for setting a current weight as a current value, a target weight as a target value, and a period or a time limit for attaining the target value displayed in the screen of the controller illustrated in Fig. 2.
Fig. 9 is a front view illustrating another example of the screen for setting a current weight as a current value, a target weight as a target value, and a period or a time limit for attaining the target value displayed in the screen of the controller illustrated in Fig. 2.
Fig. 10 is a front view illustrating another example of the screen for inputting user related information displayed in the screen of the controller illustrated in Fig. 2.
Fig. 11 is a front view illustrating an example of a screen for a plurality of choices displayed in the screen of the controller illustrated in Fig. 2.
Fig. 12 is a front view illustrating an example of a screen for a current value and target information displayed in the screen of the controller illustrated in Fig. 2.

### BEST MODE FOR CARRYING OUT THE INVENTION

### Configuration of Treatment System]

Hereinafter, the overall configuration of a treatment system 1 according to an embodiment of the present invention will be described.

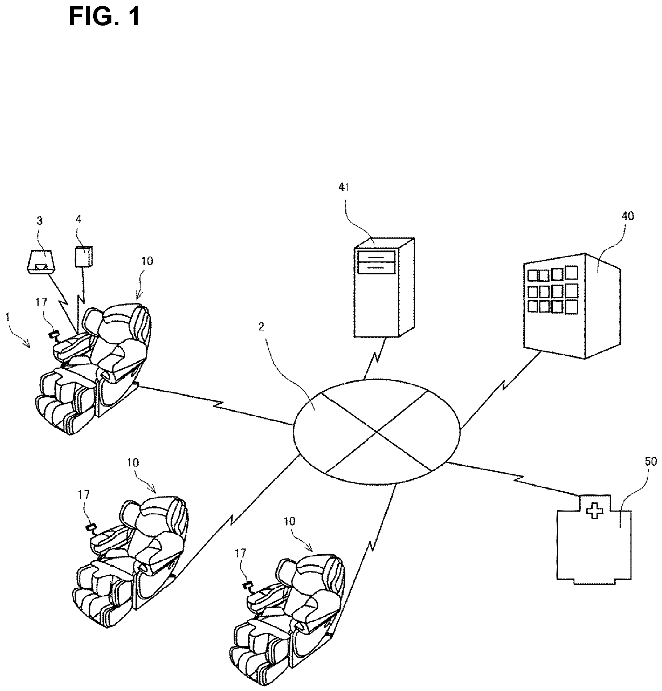

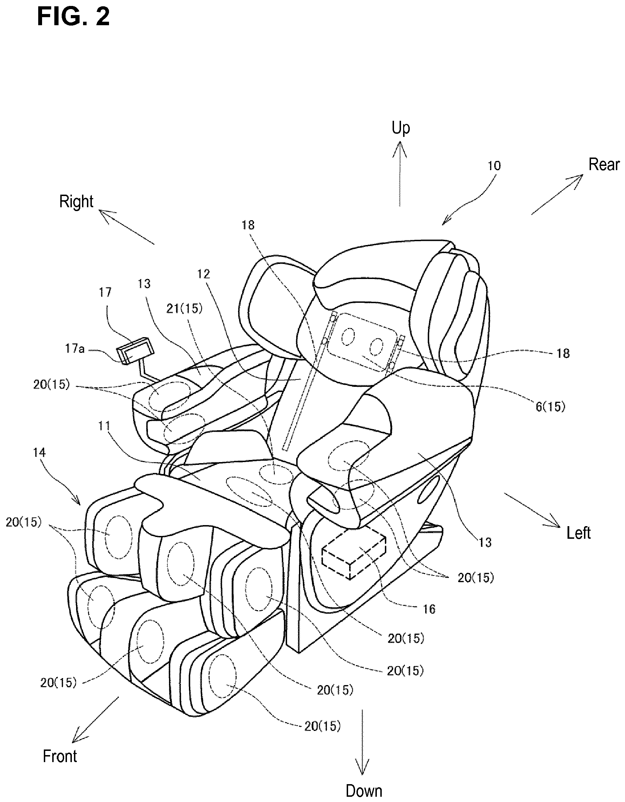

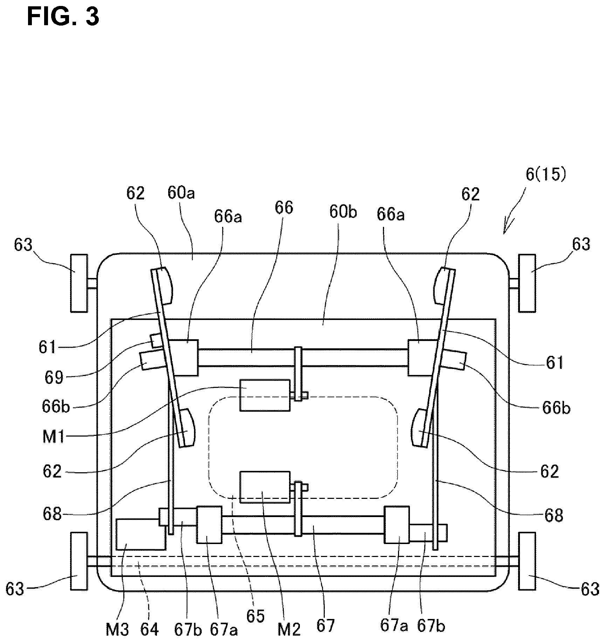

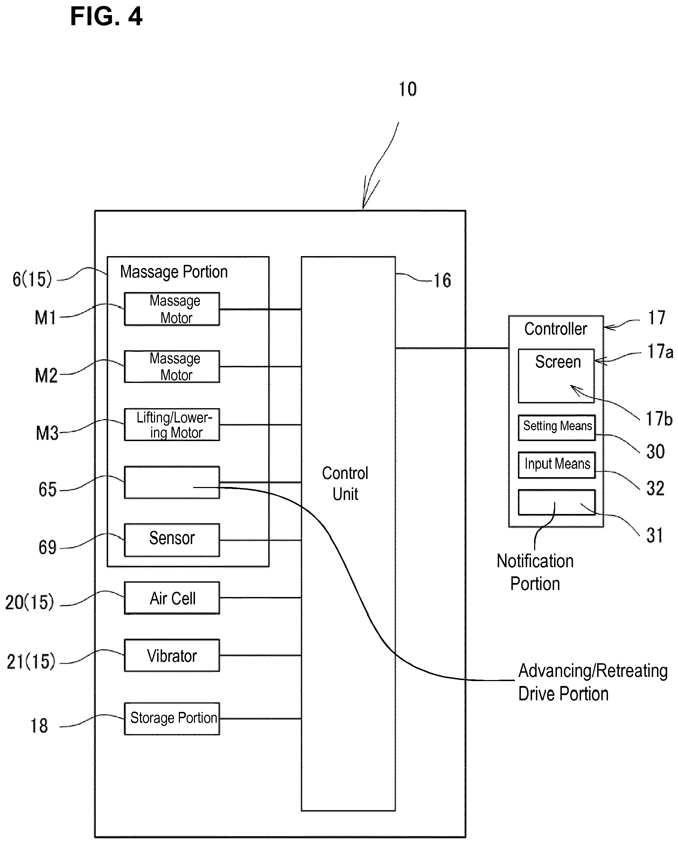

Fig. 1 is a view of the overall configuration of the treatment system 1 according to the embodiment of the present invention. Fig. 2 is a perspective front view of a massage machine 10 illustrated in Fig. 1. Fig. 3 is a front view of a massage unit 6. Fig. 4 is a block diagram of the massage machine 10. The concept of directions used in the description below coincides with the concept of directions when viewed from a user using the massage machine 10 illustrated in Fig. 2, and other cases will be suitably described. In addition, as the definition of the front and the back of the body, "front surface" indicates the chest side of the user who is standing, and "back surface" indicates the back side.

As illustrated in Fig. 1, the treatment system 1 according to the embodiment of the present invention includes the massage machine 10 which massages the user, and a weight scale 3 which takes weight information of the user. In addition, the weight scale 3 may be substituted by a body composition meter (not illustrated) which can take a body fat percentage or a body fat quantity. The weight scale 3 can communicate with the massage machine 10 through radio communication or cable communication. In addition, the weight scale 3 may be built in the massage machine 10.

The treatment system 1 is connected to a communication network 2. A communication server 41 can communicate with the massage machine 10 via the communication network 2. For example, examples of the communication network 2 include the internet network, a communication network in a building, and a local network.

In addition, the communication server 41, a service provider 40, and a hospital (medical facility) 50 are connected to the communication network 2. In this example, an example of a cloud-type communication server 41 is illustrated. The communication server 41 may be a communication server 41 managed by the service provider 40 or may be a communication server 41 managed by a different service provider 40. For example, examples of the service provider 40 include a service provider 40 providing rental service of the massage machine 10, and a service provider 40 managing the massage machine 10 which has been sold.

For example, examples of the hospital 50 include a hospital 50 that is affiliated with the service provider 40 and provides advice related to health based on body information such as the weight of the user. The hospital 50 and the service provider 40 may be one body. The service provider 40 and the hospital 50 are not necessarily connected to each other via the communication network 2. According to the configuration including the hospital 50, the service provider 40 can provide the hospital 50 with the body information of the user. The hospital 50 can provide the user with advice based on the body information of the user. The advice can be transmitted from the service provider 40 to the massage machine 10 via the communication network 2 and can be provided for the user.

In addition, the weight scale 3 can communicate with the massage machine 10, and an information terminal 4 recording the weight information of the user may be additionally included therebetween. As the information terminal 4, a tablet terminal, a portable telephone, a wearable terminal, or the like can be used. The information terminal 4 is configured to be able to communicate with the massage machine 10 through cable communication or radio communication.

In such a configuration, the user can check his/her own weight information through the information terminal 4 even at a place away from the massage machine 10. The massage machine 10 receives the weight information of the user through the information terminal 4 and can set the weight information of the user, as a current value (will be described later).

### Configuration of Massage Machine

As illustrated in Figs. 2 to 4, the massage machine 10 has a seat portion 11 in which the user takes a seat, and a backrest portion 12 which supports the upper half of the body of the user who takes a seat in the seat portion 11, from the back. The seat portion 11 has armrests 13 at the right and left positions. The seat portion 11 has a footrest 14 in the front of the seat portion 11. In addition, the massage machine 10 has a massage portion 15 which performs a massage with respect to the user, a control unit 16 which controls various types of operations of the massage machine 10, and a controller 17 which allows the user to perform various types of operations.

As the massage portion 15, there is provided at least one of the massage unit 6 which has treatment members 62 making a pair on the right and left and being driven by motors M1 to M3, an air cell 20 which expands and contracts in response to air supply/exhaust, and a vibrator 21 which is driven by a motor. In the present embodiment, the massage unit 6 is provided in the backrest portion 12, the air cell 20 or the vibrator 21 is provided in the seat portion 11, and the air cell 20 or the vibrator 21 is provided in the armrests 13 or the footrest 14. However, disposition of each massage portion 15 is not limited thereto. The air cell 20 can press the user through air supply/exhaust. The vibrator 21 can apply vibration to the user by rotating an eccentric weight portion.

### Configuration of Massage Unit

As illustrated in Fig. 3, the backrest portion 12 is provided with the massage unit 6 which massages the upper half of the body of the user from the back (back surface). The massage unit 6 is configured to include arms 61 making a pair on the right and left, and the treatment members 62 provided in both upper and lower end portions in each arm 61. The massage unit 6 can perform a kneading massage in which the treatment members 62 on the right and left approach each other and are separated from each other in response to driving of the massage motor M1, and a patting massage in which the treatment members 62 on the right and left alternately advance and retreat with respect to the user side in response to driving of the massage motor M2. In addition, the massage unit 6 moves upward or downward along the height direction in response to driving of the lifting/lowering motor M3. Accordingly, the massage unit 6 can change the position with respect to the body or can perform a rolling massage. In the backrest portion 12, there are provided guide rails 18 making a pair on the right and left and extending in the height direction (refer to Fig. 2), and the massage unit 6 moves along the guide rails 18. Since the massage unit 6 can move in the height direction, the user can be massaged by the treatment members 62 from the neck to the waist.

As illustrated in Fig. 3, the massage unit 6 has a base frame 60a and a movable frame 60b which is supported by the base frame 60a. The base frame 60a has guide rollers 63 which are fitted to both the right and left sides of the guide rails 18. The base frame 60a can be moved along the height direction by a lifting/lowering mechanism (not illustrated) formed with rack and pinion, or the like. The movable frame 60b is supported by the base frame 60a via an oscillation axis 64 in the lateral direction. Between the base frame 60a and the movable frame 60b, there is provided an advancing/retreating drive portion 65 formed with an air cell or the like. In response to driving of the advancing/retreating drive portion 65, the movable frame 60b can advance and retreat with respect to the user while having the oscillation axis 64 as the center. It is not compulsory to have the structure in which the movable frame 60b advances and retreats. A structure in which the arms 61 are respectively provided with the advancing/retreating drive portions 65 and only the arms 61 advance and retreat may be adopted.

The arms 61 are interlocked with a kneading shaft 66 and a patting shaft 67 extending in the lateral direction. On both the right and left sides of the kneading shaft 66, there are respectively provided bevel cams 66a having bevel shaft portions 66b. The arms 61 are respectively attached to the bevel cams 66a. The bevel shaft portions 66b on the right and left are beveled with respect to the shaft center of the kneading shaft 66 so as to form a substantially inverted V-shape in a front view. On both the right and left sides of the patting shaft 67, there are respectively provided eccentric cams 67a having eccentric shaft portions 67b which are eccentric with respect to the shaft center of the patting shaft 67. The arms 61 are respectively attached to the eccentric cams 67a via the connecting rods 68. In the eccentric shaft portions 67b on the right and left, the phases with respect to the shaft center of the patting shaft 67 are different from each other. Specifically, the phases are different from each other as much as 180 degrees. The kneading shaft 66 and the patting shaft 67 respectively rotate in response to driving of the massage motors M1 and M2. The treatment members 62 perform a kneading massage in response to rotation of the kneading shaft 66 and perform a patting massage in response to rotation of the patting shaft 67. The arms 61 provided with the treatment members 62, the kneading shaft 66, and the patting shaft 67 are supported by the movable frame 60b. Therefore, the treatment members 62 can advance and retreat with respect to the user via movement of the movable frame 60b.

The arms 61 oscillate freely in the forward/backward direction, and the upper treatment members 62 are biased by biasing means (not illustrated) formed with a spring or the like so as to protrude forward. In addition, the massage unit 6 has a sensor 69 which detects the body information of the user. The sensor 69 can acquire the body information by detecting that the arms 61 are at predetermined oscillation positions. Specifically, in a process of lifting the massage unit 6 along the height direction, when the upper treatment members 62 reach upper portions of the shoulder, loads acting on the treatment members 62 are cancelled, and the arms 61 oscillate forward due to action of the biasing means (not illustrated), thereby being at predetermined oscillation positions. The sensor 69 detects that the arms 61 are at predetermined oscillation positions. The sensor 69 detects the position of the shoulder based on the vertical position of the massage unit 6 at that time. Based on the position of the shoulder, positions of other sites (neck, back, waist, and the like) are obtained through calculation. The detected body information is stored in a storage portion 18 (will be described later) by the control unit 16 (will be described later). The user may directly input the body information. In this case, for example, the body information such as height information of the user can be input by operating the controller 17, and the input body information can be stored in the storage portion 18 by the control unit 16.

### Configuration of Control Unit

As illustrated in Fig. 4, the control unit 16 has a programmable microcomputer and the like, and the control unit 16 drives and controls the massage portion 15. Each of the motors M1 to M3, the advancing/retreating drive portion 65, and the sensor 69 configuring the massage unit 6; the massage portion 15 configured to include the massage unit 6, the air cell 20, the vibrator 21, or the like; the controller 17 operated by the user; and the storage portion 18 are electrically connected to the control unit 16. In addition, in the storage portion 18, the weight information of the user from the weight scale 3 is chronologically recorded by the control unit 16. For example, as the storage portion 18, a flash memory, a hard disk drive, or the like may be used.

The massage machine 10 is operated in accordance with a preset program (massage course) stored in the storage portion 18 and is also operated in accordance with an instruction of the user given through the controller 17.

The controller 17 is provided above one armrest 13. The controller 17 has a screen 17a which can be visually checked by the user, and a touch panel 17b which is operated by the user using a fingertip. A massage operation performed by the massage portion 15 can be operated through the controller 17. The controller 17 is provided so as to be able to be operated in a state where the user takes a seat. The controller 17 functions as setting means 30 for setting a numerical value including weight information of the user to be set as a target value (will be described later), a notification portion 31, and input means 32 for inputting user related information (will be described later). By performing an operation through the touch panel 17b, the numerical value including the weight information of the user can be set and the user related information can be input.

A different unit other than the controller 17 may function as the setting means 30, the notification portion 31, and the input means 32. For example, the information terminal 4 which is separate and independent from the massage machine 10, is able to perform radio communication or cable communication, and records the weight information of the user may be adopted. In addition, the notification portion 31 can issue a notification through a voice or flashing of an LED or the like, in addition to displaying a screen.

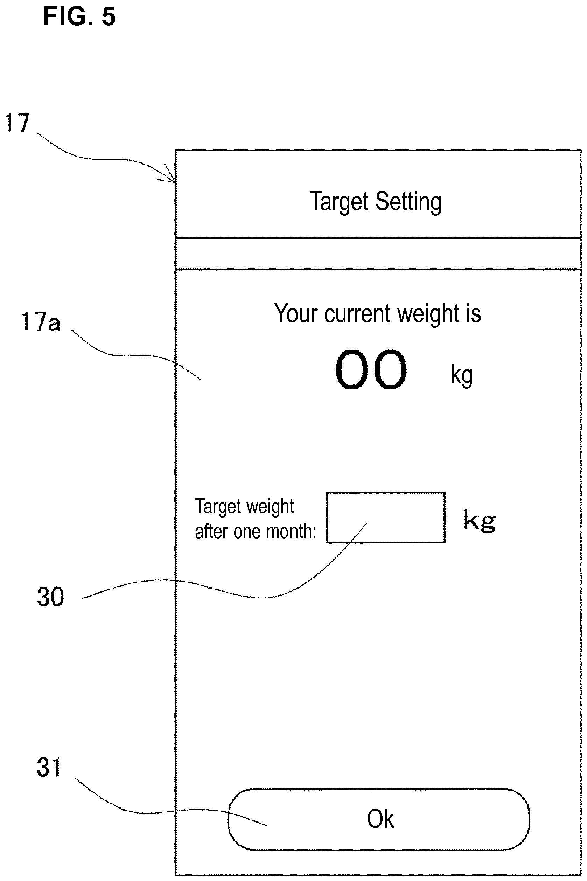

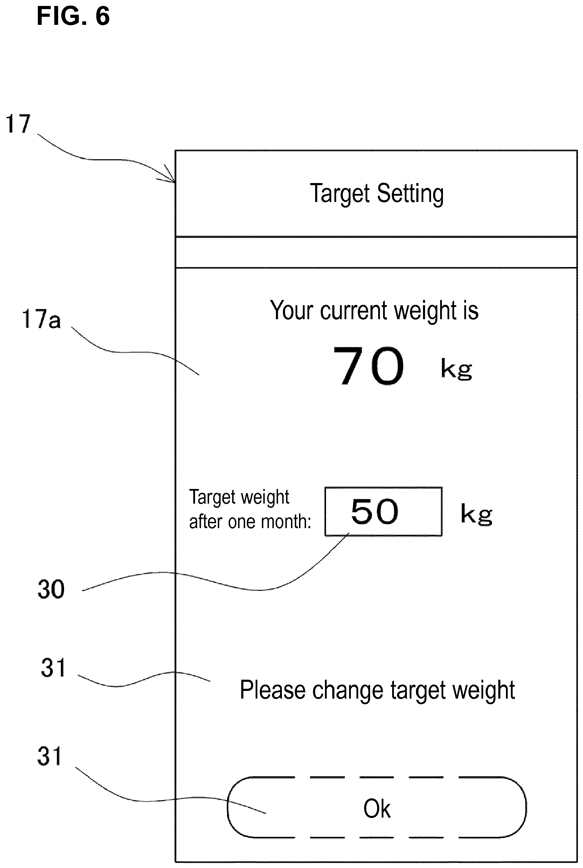

Figs. 5 and 6 are front views illustrating examples of a screen for setting a target weight as a target value displayed in the screen 17a of the controller 17. In this example, the control unit 16 receives a result of a measurement taken through the weight scale 3 by the user, and the result is displayed in an upper portion of the screen 17a of the controller 17. In addition, in place of the measurement taken through the weight scale 3, the screen 17a may display a setting screen in which a current weight can be set, and the current weight may be set through the setting means 30. Below thereof, a numerical value of the target weight as a target value by the user can be set through the setting means 30. In this example, the screen is displayed so as to set a target weight after one month. In addition, the notification portion 31 is provided in a lower portion of the screen. The control unit 16 compares the target value and a predetermined reference value with each other. In a case where a predetermined condition is satisfied, the control unit 16 can cause the notification portion 31 to urge the user to change the target value and can prohibit the target value from being set.

As illustrated in Fig. 6, for example, as a result of a measurement taken through the weight scale 3 by the user, the current weight of 70 kg is displayed. In a case of setting the target weight as the target value to 50 kg, the user sets 50 kg through the setting means 30. For example, when the control unit 16 determines that the weight which may be reduced in one month is 10 kg, the predetermined reference value becomes 60 kg by subtracting 10 kg from 70 kg. The control unit 16 compares the target value of 50 kg and the reference value of 60 kg. In a case where a predetermined condition such as "the target value is less than 60 kg" is satisfied, the control unit 16 can cause the notification portion 31 to display "please change the target weight" in the screen and to urge the user to change the target weight. The control unit 16 can hinder an "OK" button of the notification portion 31 from being pressed and can prohibit the user from setting the target weight. The predetermined condition is not limited to the above-described example. In addition, the user may be urged to change the target weight through a notification by outputting a voice instead of displaying a screen with a text message. The contents of the notification are not limited to the above-described example. The user can press the "OK" button of the notification portion 31 by resetting the target weight to 60 kg through the setting means 30, and the user can proceed to the next step by pressing the "OK" button. In this manner, the user can set an appropriate target value instead of an immoderate target value.

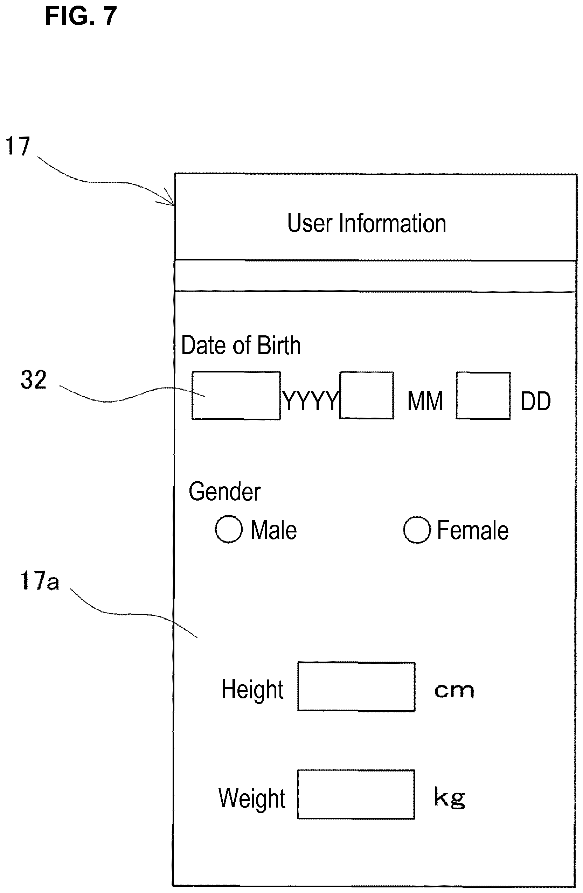

Fig. 7 is a front view illustrating an example of a screen for inputting user related information displayed in the screen 17a of the controller 17. In this example, the screen 17a of the controller 17 displays the input means 32 for inputting user related information. The date of birth, the gender, the height, and the weight of the user can be input through the input means 32. The control unit 16 can set the reference value based on the user related information input through the input means 32. For example, in a case where October 8, 1980 as date of birth, male, 170 cm, and 70 kg are input, the reference value becomes a standard weight of 63.6 kg calculated based on the height of 170 cm and the BMI of 22. If the gender is female, the reference value becomes a standard weight of 60.7 kg calculated based on the height of 170 cm and the BMI of 20.8. In this manner, the reference value can be set based on the user related information. The method of setting the reference value is not limited to the above-described example.

In the examples illustrated in Figs. 5 and 6, the numerical value including the weight information of the user to be set as the target value is the weight. However, the numerical value includes the BMI and the body fat percentage or the body fat quantity, and the predetermined reference value also includes the BMI and the body fat percentage or the body fat quantity. In addition, the predetermined reference value may be set with a value of the standard weight or a general standard value. For example, 22 for the BMI, 20% for the body fat percentage, and the value of weight×20% of body fat percentage for the body fat quantity are set as the reference value. The predetermined condition for comparing the target value and the reference value with each other requires that the target value is less than the reference value. In this manner, according to the predetermined condition having the target value to be less than the reference value, the user can set a more appropriate target value. The predetermined condition is not limited to the above-described example.

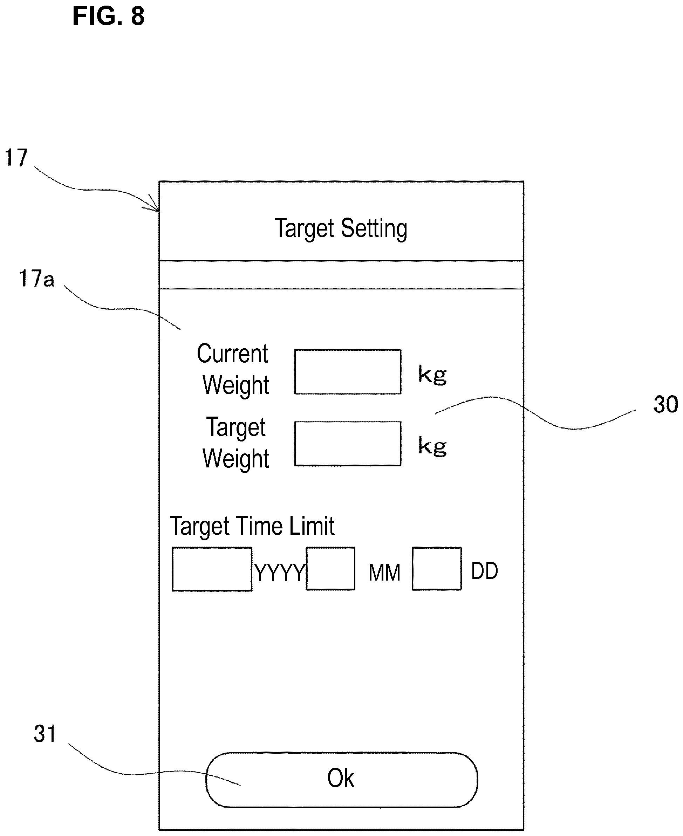

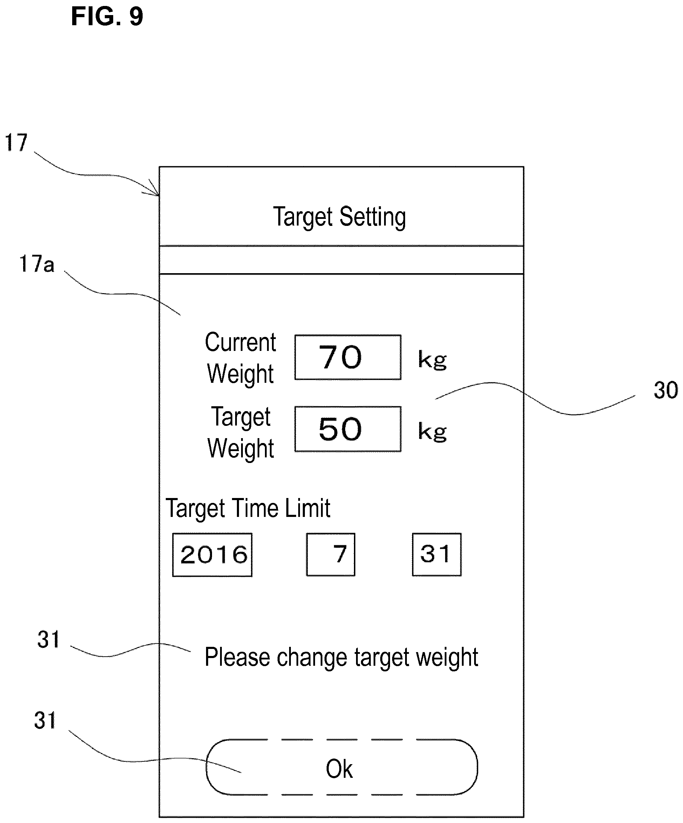

Figs. 8 and 9 are front views illustrating examples of a screen for setting a current weight as a current value, a target weight as a target value, and a period or a time limit for attaining the target value displayed in the screen 17a of the controller 17. In this example, the numerical value of the current weight as the current value, the numerical value of the target weight as the target value, and the period or the time limit for attaining the target value can be set by the user through the setting means 30. In addition, the notification portion 31 is provided in a lower of the screen 17a. In a case where a relationship between the difference of the current value and the target value, and the period or the time limit satisfies the predetermined condition, the control unit 16 can cause the notification portion 31 to urge the user to change at least one of the target value and the period or the time limit or can prohibit at least one of the target value and the period or the time limit from being set.

As illustrated in Fig. 9, for example, in a case where the user sets the current weight as the current value to 70 kg, sets the target weight as the target value to 50 kg, and sets the target time limit as the period or the time limit to July 31, 2016 through the setting means 30, and in a case where the relationship between the difference of the current value and the target value, and the period or the time limit satisfies a predetermined condition such as "the weight to be reduced in one month is equal to or less than 5%", the control unit 16 causes the notification portion 31 to display "please change the target weight" in the screen and to urge the user to change the target weight. The control unit 16 hinders the "OK" button of the notification portion 31 from being pressed and prohibits the user from setting the target weight or the target time limit. The predetermined condition is not limited to the above-described example. A condition such as "the weight to be reduced in one month is equal to or less than 5 kg" may be adopted. In addition, the user may be urged to change the target weight through a notification by outputting a voice instead of displaying a screen with a text message. The contents of the notification are not limited to the above-described example. The user can press the "OK" button of the notification portion 31 by resetting the target weight to 60 kg through the setting means 30, and the user can proceed to the next step by pressing the "OK" button. The method of urging the user to make a change is not limited to the above-described example. The user may be urged to change the target time limit for the period or the time limit. In addition, the method of prohibiting the target weight or the target time limit from being set is not limited to the above-described example. The method is acceptable even if at least one of the target weight and the target time limit is only prohibited from being set. In this manner, the user can set an appropriate target value and an appropriate period or an appropriate time limit instead of an immoderate target value and an immoderate period or an immoderate time limit.

In the examples illustrated in Figs. 8 and 9, the current value and the numerical value including the weight information of the user to be set as the target value is the weight. However, the numerical values include the BMI and the body fat percentage or the body fat quantity. The predetermined condition requires that an average change rate calculated based on the difference of the current value and the target value, and the period or the time limit exceeds a predetermined change rate. As the predetermined change rate, for example, a predetermined change rate for the weight is set such as "the weight to be reduced in one month is 5%", a predetermined change rate for the BMI is set such as "the BMI is reduced by 1 in one month", and a predetermined change rate for the body fat percentage is set such as "reduced by 1% in one month". The predetermined change rate is not limited to the above-described example. In this manner, the user can set an appropriate target value and an appropriate period or an appropriate time limit and can plan an ideal schedule.

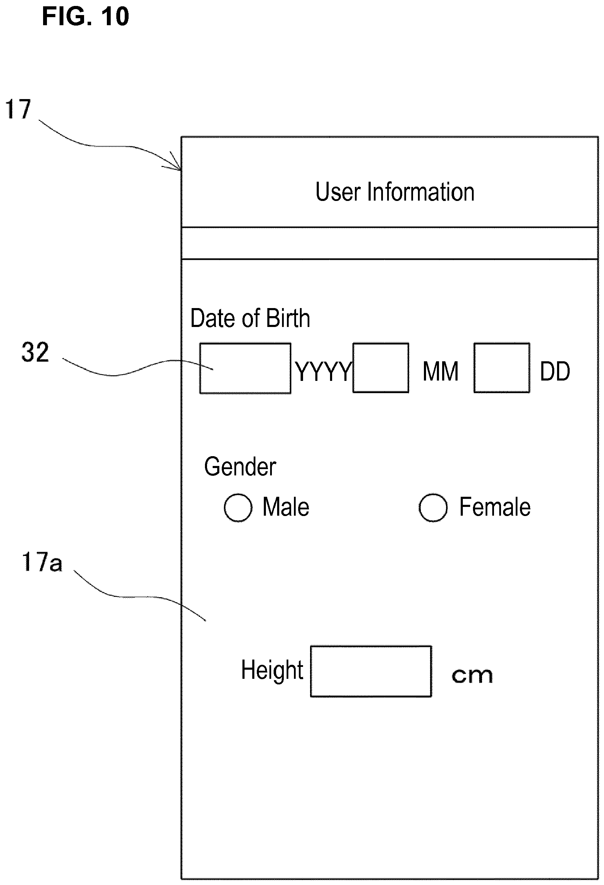

Fig. 10 is a front view illustrating another example of the screen for inputting user related information displayed in the screen 17a of the controller 17. In this example, the screen 17a of the controller 17 displays the input means 32 for inputting user related information. The date of birth, gender, and the height of the user can be input through the input means 32. The control unit 16 can set a change rate based on the user related information input through the input means 32. For example, in a case where October 8, 1980 is input as date of birth, male is input as the gender, and 170 cm is input as the height, the predetermined change rate for the weight of the user is set such as "the weight to be reduced in one month is 5%". In addition, if the gender is female, the predetermined change rate is set such as "the weight to be reduced in one month is 3%". In this manner, the change rate can be set based on the user related information. The change rate is not limited to the above-described example.

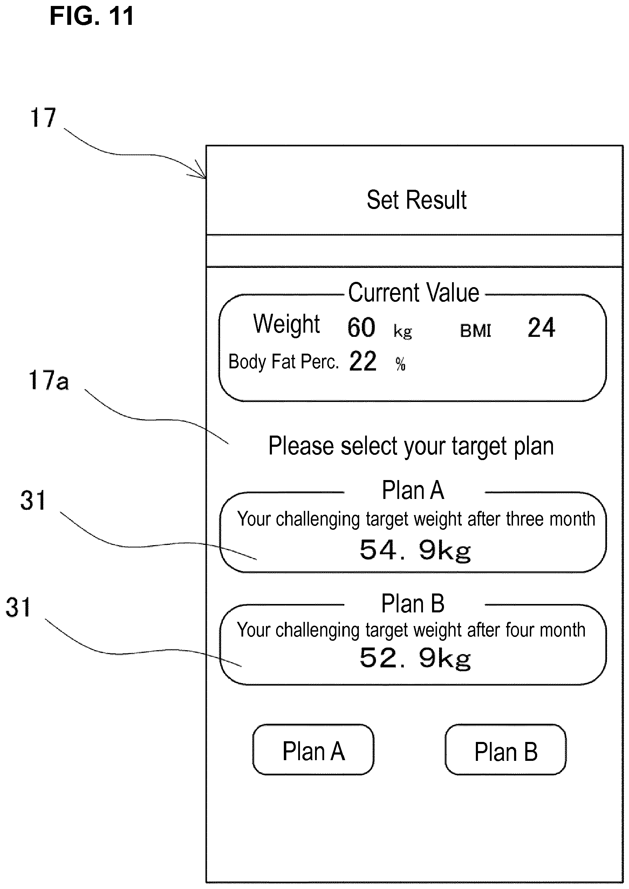

Fig. 11 is a front view illustrating an example of a screen for a plurality of choices displayed in the screen 17a of the controller 17. In this example, description will be given regarding an example in which the user related information is input in advance through the input means 32 and a current numerical value including the weight information of the user to be set as the current value is set through the setting means 30. The control unit 16 causes the screen 17a to display the information input by the user, and the current numerical value including the weight information of the user to be set as the current value. The control unit 16 calculates a plurality of target values and a plurality of periods or a plurality of time limits for attaining the target values based on the user related information and the current value, draws out a plurality of choices constituted by the plurality of target values and the plurality of periods or the plurality of time limits, and causes the notification portion 31 to issue a notification.

As illustrated in Fig. 11, for example, as the information input through the input means 32 and the result set through the setting means 30, the screen 17a displays the weight of 60 kg, the BMI of 24, and the body fat percentage of 22% as the current values, and two types of target plans are displayed for the user based on the current values. In two types of the target plans, for Plan A, the target weight of 54.9 kg in a period of three months is displayed. For Plan B, the target weight of 52.9 kg in a period of four months is displayed. The user can make a selection from two types of the target plans.

In this manner, by utilizing the user related information input by the user and the numerical value of the weight information set as the current value, a plurality of target plans are displayed together with the target value and the period or the time limit. Thus, the user can set a target suitable for himself/herself from the plurality of choices.

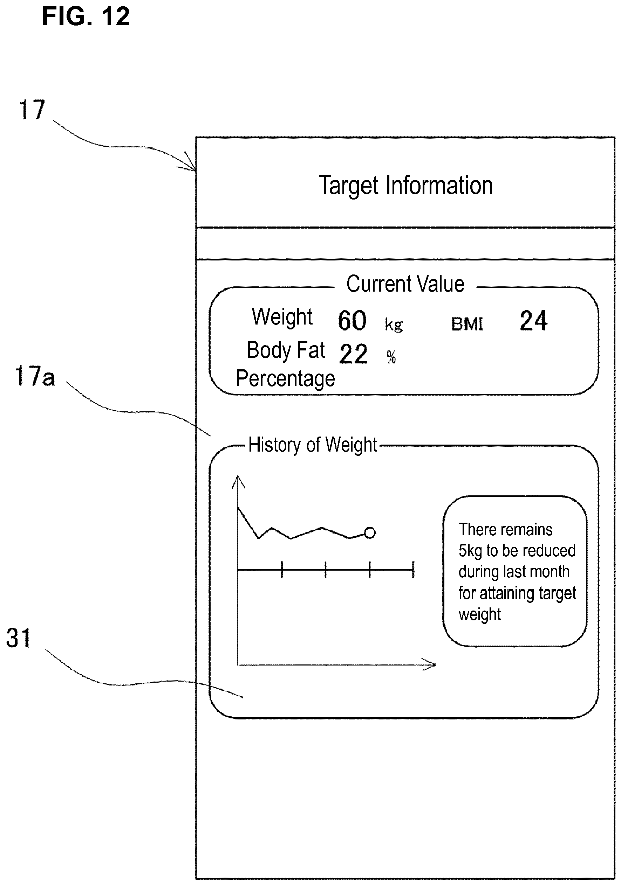

Fig. 12 is a front view illustrating an example of a screen for a current value and target information displayed in the screen 17a of the controller 17. In this example, description will be given regarding an example in which a value at the time of being set by the user, the target value, and the period or the time limit for attaining the target value are set in advance and a current value is obtained based on the target value. The target information indicating the relationship among the current value, the target value, and the period or the time limit for attaining the target value can be notified through the notification portion 31. The control unit 16 causes the screen 17a of the controller 17 to display the current value in an upper portion thereof and causes the screen 17a to graphically display the chronological change of the weight of the user in the notification portion 31 at the middle thereof. The graphic display is prepared based on the target information indicating the relationship among the current value, the target value, and the period or the time limit for attaining the target value. In addition, the target information may be notified by the voice.

As illustrated in Fig. 12, for example, the user sets the target time limit to four months, and the weight tends to be favorably reduced during the first month. However, it is found that the weight repeats to increase and decrease at the present stage, that is, during the second to third months. There remains 5 kg to be reduced during the last month for attaining the target weight.

In this manner, by utilizing the current value of the user, the change and the current circumstances of the weight of the user are displayed. Accordingly, the controller 17 can notify the user of the change of his/her own weight and the target information by displaying a graph or outputting a voice. In Fig. 12, monthly history of the weight is displayed. However, the display may be switched to detailed display such as weekly or daily history. In this manner, the user can know the current circumstances in more detail.

### Another Embodiment

In addition, the treatment system 1 of the present invention is not limited to the illustrated form and may adopt a different form within the scope of this invention.

For example, in the above-described embodiment, a weight scale is used as an example. However, a measuring instrument such as an activity meter may be adopted. The activity meter takes not only the number of steps but also various activities such as household affairs and desk work and allows the user to see the daily total calorie consumption. By being connected to the massage machine 10, the screen 17a of the controller 17 can display the current circumstances with a numerical value or a graph. In addition, an appropriate target can be set and can be help of health management.

### INDUSTRIAL APPLICABILITY

The present invention can be applied to a treatment system including a massage machine in which when setting a target value related to a weight, a user can set an appropriate target value instead of setting an immoderate target value.

### LIST OF REFERENCE NUMERALS

- 1: TREATMENT SYSTEM
- 6: MASSAGE UNIT (MASSAGE PORTION)
- 10: MASSAGE MACHINE
- 15: MASSAGE PORTION
- 16: CONTROL UNIT
- 20: AIR CELL (MASSAGE PORTION)
- 21: VIBRATOR (MASSAGE PORTION)
- 30: SETTING MEANS
- 31: NOTIFICATION PORTION
- 32: INPUT MEANS

## Claims

1. A treatment system including a massage machine which has a massage portion performing a massage with respect to a user, and a control unit controlling the massage portion, the treatment system comprising:
setting means for setting a numerical value which includes weight information of the user to be set as a target value; and
a notification portion,
wherein the control unit compares the target value and a predetermined reference value with each other and causes the notification portion to urge the user to change the target value in a case where a predetermined condition is satisfied.

2. The treatment system including a massage machine, according to Claim 1,
wherein the control unit compares the target value and the predetermined reference value with each other and prohibits the set target value from being set in a case where the predetermined condition is satisfied.

3. The treatment system including a massage machine, according to Claim 1 or 2, further comprising:
input means for inputting user related information,
wherein the reference value is set based on the user related information.

4. The treatment system including a massage machine, according to any one of Claims 1 to 3,
wherein the numerical value is a weight, a BMI, and a body fat percentage or a body fat quantity, and
wherein the predetermined condition requires that the target value is less than the reference value.

5. A treatment system including a massage machine which has a massage portion performing a massage with respect to a user, and a control unit controlling the massage portion, the treatment system comprising:
setting means for setting a current numerical value which includes weight information of the user to be set as a current value, a targeted numerical value which includes weight information of the user to be set as a target value, and a period or a time limit for attaining the target value; and
a notification portion,
wherein the control unit causes the notification portion to urge the user to change at least one of the target value and the period or the time limit in a case where a relationship between a difference of the current value and the target value, and the period or the time limit satisfies a predetermined condition.

6. The treatment system including a massage machine, according to Claim 5,
wherein the control unit prohibits at least one of the target value and the period or the time limit from being set in a case where the relationship between the difference of the current value and the target value, and the period or the time limit satisfies the predetermined condition.

7. The treatment system including a massage machine, according to Claim 5 or 6,
wherein the numerical value is a weight, a BMI, and a body fat percentage or a body fat quantity, and
wherein the predetermined condition requires that an average change rate calculated based on the difference of the current value and the target value, and the period or the time limit exceeds a predetermined change rate.

8. The treatment system including a massage machine, according to Claim 7, further comprising:
input means for inputting user related information,
wherein the change rate is set based on the user related information.

9. A treatment system including a massage machine which has a massage portion performing a massage with respect to a user, and a control unit controlling the massage portion, the treatment system comprising:
input means for inputting user related information;
setting means for setting a current numerical value which includes weight information of the user to be set as a current value; and
a notification portion,
wherein the control unit calculates a plurality of target values and a plurality of periods or a plurality of time limits for attaining the target values based on the information and the current value, draws out a plurality of choices constituted by the plurality of target values and the plurality of periods or the plurality of time limits, and causes the notification portion to issue a notification.

10. The treatment system including a massage machine, according to any one of Claims 5 to 9,
wherein the notification portion issues a notification of target information indicating a relationship among the current value, the target value, and the period or the time limit.
